# EUROPÄISCHE PATENTSCHRIFT

(11) **EP 1 134 279 B1**
(45) Veröffentlichungstag und Bekanntmachung des Hinweises auf die Patenterteilung: **01.06.2005**
(21) Anmeldenummer: 01104096.1
(22) Anmeldetag: 21.02.2001
(51) Int. Cl.: C11D 3/48, C11D 1/62, C11D 3/20, A01N 37/20, A01N 31/14, A61K 7/50, A61K 7/48, A61L 2/18

(54) **Mikrobizide Wirkstoffkombinationen**
Antimicrobial active substance combinations
Association de principes actifs antimicrobienne

(30) Priorität: 14.03.2000 DE 10012338
(43) Veröffentlichungstag der Anmeldung: 19.09.2001
(73) Patentinhaber: Bode Chemie GmbH & Co., 22525 Hamburg (DE)
(72) Erfinder: Rudolf, Marco, Dr., 22299 Hamburg (DE); Knieler, Roland, Dr., 22529 Hamburg (DE); Ostermeyer, Christiane, 22559 Hamburg (DE); Lackner, Sibylle, 22159 Hamburg (DE)
(74) Vertreter: Rabanus, Birgit

(56) Entgegenhaltungen:
- EP-A- 0 872 230
- WO-A-91/12721

## Beschreibung

Die vorliegende Erfindung betrifft mikrobizide Wirkstoffkombinationen mit guter Hautund Schleimhautverträglichkeit sowie Desinfektionsmittel und Antiseptika, dieselben enthaltend, insbesondere Desinfektionsmittel und Antiseptika zur Behandlung der Hände und der Haut. Femer betrifft die vorliegende Erfindung Waschlotionen (Flüssigseifen) oder auch feste Seifen zur antiseptischen Ganzkörperwaschung, zur antiseptischen Haarwäsche, zur antiseptischen Hand- und/oder Fußwäsche, zur antiseptischen Intimwäsche oder zur Desinfektion von Händen, Haut und Schleimhäuten.

Der gesunde warmblütige Organismus, insbesondere die gesunde menschliche Haut, ist ständig mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Durch normales Waschen mit Wasser und Seife wird die Keimzahl der Hautoberfläche auf ein Hundertstel bis auf ein Tausendstel (entsprechend einem logarithmischen Reduktionsfaktor von 2 bis 3) reduziert. Für die normale Körperhygiene ist diese Keimreduktion gewünscht und ausreichend. Eine zusätzliche antimikrobielle Ausrüstung ist dementsprechend normalerweise nicht erforderlich; allerdings gibt es Fälle, wo ein antimikrobiell wirksames Waschmittel erwünscht, sinnvoll oder sogar erforderlich ist. Im Folgenden werden einige Beispiele aufgelistet:
- Haarpflege, insbesondere bei starkem Schuppenbefall, z. B. verursacht durch Marcecia furfur (Pythosporum ovale),
- Körperpflege bei starkem Körpergeruch, verursacht durch die Zersetzung von Schweiß durch Hautbakterien,
- im Bereich der Intimhygiene zur Infektionsprophylaxe,
- im Bereich der Fußpflege zur Fußpilzprophylaxe bzw. Fußpilztherapie und
- im Bereich der Infektionsprophylaxe im Krankenhaus gegen Befall durch Bakterien, Pilze und Viren sowie antibiotika-resistente Keime.

Die Anforderungen an antimikrobielle Wirkstoffe hinsichtlich Wirksamkeit und Verträglichkeit sind sehr hoch und werden eingehend beschrieben in *A. Kramer, D. Gröschel, P. Heeg, V. Hingst, H. Lippert, M. Rotter und W. Weuffen: "Klinische Antiseptik*", *Springer Verlag, Berlin, Heidelberg, New York, 1993, S. 23 ff, S 121 ff.* Nach den Prüfrichtlinien der Deutschen Gesellschaft für Hygiene und Mikrobiologie (*Zbl. Bakt. Hyg. B 182, 562-570, 1987*) soll die Keimreduktion für antiseptische Händereinigungsmittel (Händedekontaminationsmittel) 3,5 log-Stufen (entsprechend 99,9684 %) innerhalb von 30 Sekunden betragen. Als Testkeime werden Staphylococcus aureus, Escherichia coli, Proteus mirabilis Pseudomonas aeruginosa sowie Candida albicans stellvertretend für das gesamte Keim- und Pilzspektrum verwendet.

Eine vergleichbare Europanorm (prEN 12054) ist in Vorbereitung. Hier werden als Testkeime Staphylococcus aureus, Escherichia coli, Pseudomonas aeruginosa sowie Enterococcus hirae vorgeschlagen. Um dieser Norm gerecht zu werden, müssen die Produkte in einem quantitativen Suspensionsversuch in einer 50 %-igen Verdünnung einen Reduktionsfaktor von 3 log-Stufen innerhalb von 1 Minute - oder vorzugsweise innerhalb von 30 Sekunden - erreichen. In einer weitergehenden Prüfung müssen die Produkte zur hygienischen Händewaschung im praxisnahen Test darüber hinaus wirksamer sein, als einfache Schmierseife, um den Anforderungen nach der Europäischen Prüfnorm EN 1499 zu genügen. Dieser Maßstab gilt nicht nur für die antiseptische Händewaschung, sondern wird - weil weitere Prüfvorschriften fehlen - auf die Ganzkörperwaschung, aber auch auf die antiseptische Waschung einzelner Körperregionen übertragen.

Hinsichtlich der Verträglichkeit gelten bei therapeutisch eingesetzten Präparaten gelten die Grundsätze der pharmakologisch-toxikologischen Prüfungen gemäß dem Arzneimittelgesetz. *Kramer und Wallhäußer* (in: *A. Kramer, D. Gröschel, P. Heeg, V. Hingst, H. Lippert, M. Rotter und W. Weuffen: "Klinische Antiseptik*", *Springer Verlag,* *Berlin, Heidelberg, New York, 1993, S. 21 ff*.) meinen hierzu: "Bei prophylaktisch eingesetzten Produkten existieren keine standardisierten toxikologischen Prüfanforderungen, daher sind die jeweils verfügbaren Informationen zur Verträglichkeit bei der Auswahl eines Antiseptikums besonders verantwortungsbewusst zu berücksichtigen."

Von der US-amerikanischen Food and Drug Administration Behörde FDA wurde 1974 eine Liste mit Klassifikationen der gebräuchlichen antiseptischen Wirkstoffe zur prophylaktischen Anwendung auf Haut und Wunden nach den Hauptmerkmalen Wirksamkeit und Verträglichkeit vorgeschlagen (*Kramer et al*. "*Klinische Antiseptik*", *Springer Verlag, Berlin, Heidelberg, New York, 1993, S. 27*). Die dort aufgeführten ca. 15 antiseptischen Wirkstoffe Benzalkoniumchlorid, Cloflucarban, Fluorosalan, Hexylresorcinol, Methylbenzethoniumchlorid, Nonylphenoxypoly(ethylenoxy)ethanoljod, 6-Chlor-2-xylenol, Phenol, Hexachlorophen, Jodophore, Jodtinktur, Tribromsalan, Triclocarban, Triclosan, Triphenylmethanfarbstoffe stellen im wesentlichen auch heute noch den Stand der Technik dar.

Von diesen werden nur zwei - nämlich Benzalkoniumchlorid und Jodtinktur - als gleichzeitig wirksam und verträglich eingestuft, allerdings auch nur eingeschränkt. Die anderen Wirkstoffe sind entweder schlechter oder unzureichend dokumentiert oder lassen sich praktisch nicht in antiseptische Formulierungen einarbeiten, da sie physikalisch und/oder chemisch inkompatibel sind.

Weitere schon seit geraumer Zeit bekannte antimikrobielle Wirkstoffe sind unter anderen Benzoxoniumchlorid, Alkylalkohole, 2-Phenylphenol und Chlorhexidin. Allerdings haben die aufgeführten Wirkstoffe verschiedene Nachteile, die ihren Einsatz in einer antimikrobiellen Waschlotion zur Körperhygiene erschweren oder unmöglich machen.

So besitzt das 2-Phenylphenol keine ausreichende Wirksamkeit in bekannten Formulierungen, um die neuen europäischen Normen zu erfüllen. Benzoxoniumchlorid ist nur in sehr geringen Mengen in Wasser löslich und kann daher nicht in ausreichender Menge in antimikrobielle Waschlotionen etc. eingearbeitet werden, um eine entsprechende Wirksamkeit zu erreichen. Und alle Alkylalkohole weisen bei einer Konzentration von etwa 30 % ein deutliches Schleimhautreizpotential auf, was ihren Einsatz beschränkt. Chlorhexidin ist zwar schleimhautverträglich und besitzt eine ausreichende Wirksamkeit, allerdings können verschiedene gebräuchliche Waschrohstoffe eine Wirkungsverminderung hervorrufen. Des weiteren deuten viele Studien darauf hin, dass es zu einer Resistenzbildung bei dem Gebrauch von Chlorhexidin kommen kann, was bei dem Einsatz bedacht werden sollte.

Neuere Wirkstoffe sind z. B. Octenidin und Polyhexanid. Allerdings sind auch die beiden letztgenannten Wirkstoffe ungenügend. Polyhexanid ist hinsichtlich der mikrobiziden Wirksamkeit nicht ausreichend, und Octenidin lässt sich nur schwer in moderne Rezepturen einarbeiten.

Die EP-0 872 230-A1 beschreibt Haut- und Haarreinigungszubereitungen, welche neben weiteren Bestandteilen 10'-Undecen-3-oyl-aminopropyltrimethylammoniummethylsulfat enthalten, sowie ein Verfahren zur Herstellung einer solchen Haarreinigungszubereitung.

Die WO-91/12721-A1 offenbart bakterizide und virizide Desinfektionsmittel, welche auf der Haut verwendbar sind und 2,3 bis 4,0 Gew.-% 2-Phenoxyethanol; 0,8 bis 1,8 Gew.-% komplexiertes lod und 5,0 bis 20,0 Gew.-% anionische oder amphotere oberflächenaktive Substanz enthalten.

Allerdings konnte der Stand der Technik nicht den Weg zur vorliegenden Erfindung weisen.

Aufgabe der Erfindung war es somit, den Nachteilen des Standes der Technik abzuhelfen und Wirkstoffe bzw. Wirkstoffkombinationen zu finden, die hinreichend wirksam sind, dabei ein Minimum an dermatologischen Irritationspotenzial und ein Minimum an allergenem Potential aufweisen und sich leicht und mit hoher Stabilität in dermatologische Zubereitungen einarbeiten lassen.

Überraschend wurde nun gefunden und darin liegt die Lösung all dieser Aufgaben, dass Wirkstoffkombinationen aus
a) Undecylenamidopropyltrimethylammonium-Methosulfat und
b) mindestens einem Arylalkylalkohol
den Nachteilen des Standes der Technik abhelfen.

Die erfindungsgemäßen Wirkstoffkombinationen zeichnen sich durch eine gute Wirksamkeit aus, eignen sich aufgrund ihrer hohen Verträglichkeit sehr gut zur antiseptischen Ganzkörperwaschung, zur antiseptischen Haarwäsche, zur antiseptischen Hand- und/oder Fußwäsche, zur antiseptischen Intimwäsche oder zur Desinfektion von Händen, Haut und Schleimhäuten und lassen sich dabei in hervorragender Weise in Waschlotionen (Flüssigseifen), feste Seifen und ähnliche Zubereitungsformen einarbeiten.

Ein weiterer Vorteil der erfindungsgemäßen Wirkstoffkombinationen ist ihre sehr gute Haut- und Schleimhautverträglichkeit. Die Verträglichkeit bezüglich der Augenschleimhaut ist ebenfalls aussergewöhnlich gut.

Die gute Wirksamkeit war insbesondere deswegen erstaunlich, da die antimikrobielle Wirksamkeit von Arylalkoholen wie Phenoxyethanol und Phenoxypropanol normalerweise nicht den Anforderungen an ein antiseptisches Waschprodukt genügt. In Kombination mit Undecylenamidopropyltrimethylammonium-Methosulfat wird diese Wirksamkeit allerdings erreicht, offenbar wirken die Stoffe in synergistischer oder überadditiver Weise.

Undecylenamidopropyltrimethylammonium-Methosulfat zeichnet sich durch die folgende Strukturformel aus 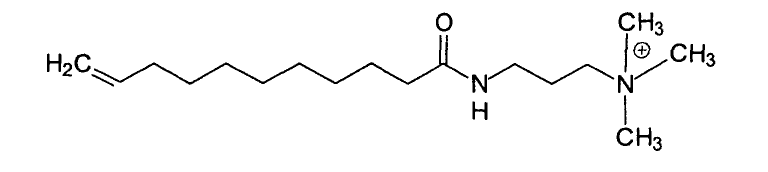 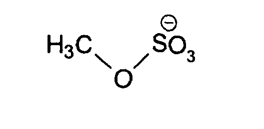 und ist beispielsweise unter der Handelsbezeichnung Rewocid UTM 185 bei der Fa. Goldschmidt Rewo erhältlich.

Vorteilhafte Arylalkyalkohole im Sinne der vorliegenden Erfindung zeichnen sich durch die folgende Strukturformel aus: 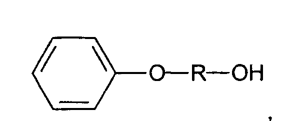 worin R eine unverzweigte oder verzweigte Alkylgruppe mit 2 bis 4 Kohlenstoffatomen darstellt. Ganz besonders vorteilhaft sind Phenoxyethanol (POE) und Phenoxypropanol (POP).

Die erfindungsgemäßen Wirkstoffkombinationen lassen sich ohne Schwierigkeiten in gängige kosmetische oder dermatologische Formulierungen einarbeiten. Die erfindungsgemäße Wirkstoffkombination ist vorteilhaft zu 0,2 bis 15 Gew.-% in einer Waschformulierung enthalten.

Vorteilhaft ist es, den Gehalt an Undecylenamidopropyltrimethylammonium Methosulfat in der Waschformulierung aus dem Bereich von 0,1 bis 10,0 Gew.-%, vorzugsweise von 2 bis 6 Gew.-% zu wählen, jeweils bezogen auf das Gesamtgewicht der Formulierungen.

Der Gehalt an einem oder mehreren Arylalkylalkoholen in der Waschformulierung wird vorteilhaft aus dem Bereich von 0,1 bis 5,0 Gew.-%, vorzugsweise von 0,5 bis 2,5 Gew.-% zu wählen, jeweils bezogen auf das Gesamtgewicht der Formulierungen. Ganz besonders vorteilhaft ist es, wenn die Zubereitungen 0,5 bis 2,5 Gew.-% Phenoxyethanol enthalten.

Dementsprechend sind auch kosmetische oder dermatologische Formulierungen, wie beispielsweise Waschlotionen, Seifen, Schmierseifen, Waschpasten, Shampoos, Duschund Badepräparate und dergleichen mehr, die eine Wirkstoffkombinationen aus Undecylenamidopropyltrimethylammonium-Methosulfat und mindestens einem Arylalkylalkohol enthalten, Gegenstand der vorliegenden Erfindung.

Kosmetische oder dermatologische Zubereitungen können neben einem wirksamen Gehalt an der erfindungsgemäßen Wirkstoffkombination weitere Stoffe enthalten, wie sie für solche Formulierungen üblich sind, wie z. B. waschaktive Substanzen, Feuchthalter, (Moisturizer), Rückfetter (Regreaser), Hautpflegestoffe, Verdicker, Farbstoffe, pH-Stellmittel, Perlglanz, Puffer, Antioxydantien, Parfums, Konservierungsmittel, weitere mikrobizide Wirkstoffe usw.

Die jeweils einzusetzenden Mengen an kosmetischen oder dermatologischen Hilfsund/oder Trägerstoffen und Parfüm können in Abhängigkeit von der Art des jeweiligen Produktes vom Fachmann durch einfaches Ausprobieren leicht ermittelt werden.

Zubereitungen gemäß der vorliegenden Erfindung können sich durch einen Gehalt an Tensiden auszeichnen. Geeignet sind im Prinzip alle anionischen, kationischen, amphoteren und nichtionischen Tenside.

Vorteilhaft zu verwendende anionische Tenside sind
Acylaminosäuren (und deren Salze), wie
   1. Acylglutamate, beispielsweise Natriumacylglutamat, Di-TEA-palmitoylaspartat und Natrium Caprylic/ Capric Glutamat,
   2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
   3. Sarcosinate, beispielsweise Myristoyl Sarcosin, TEA-lauroyl Sarcosinat, Natriumlauroylsarcosinat und Natriumcocoylsarkosinat,
   4. Taurate, beispielsweise Natriumlauroyltaurat und Natriummethylcocoyltaurat,
   5. Alaninate
Carbonsäuren und Derivate, wie
   1. Carbonsäuren, beispielsweise Laurinsäure, Aluminiumstearat, Magnesiumalkanolat und Zinkundecylenat, und Derivate, wie z. B. Acyllactylate, Lauroyllactylat, Caproyllactylat,
   2. Ester-Carbonsäuren, beispielsweise Calciumstearoyllactylat, Laureth-6 Citrat und Natrium PEG-4 Lauramidcarboxylat,
   3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
Sulfonsäuren und Salze, wie
   1. Acyl-isethionate, z. B. Natrium-/Ammoniumcocoyl-isethionat,
   2. Alkylarylsulfonate,
   3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
   4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
   sowie
Schwefelsäureester, wie
   1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
   2. Alkylsulfate, beispielsweise Natrium-, Ammonium- und TEA- Laurylsulfat.
Vorteilhaft zu verwendende kationische Tenside sind
   1. Alkylamine,
   2. Alkylimidazole,
   3. Ethoxylierte Amine und
   4. Quaternäre Tenside.
   5. Esterquats

Zubereitungen gemäß der vorliegenden Erfindung können ferner vorteilhaft weitere quaternäre Ammoniumverbindungen enthalten, insbesondere solche, welche als quaternäre Tenside bezeichnet werden. Vorteilhaft zu verwenden sind Alkylbetain, Alkylamidopropylbetain und Alkylamidopropylhydroxysulfain, Alkylamidethyltrimethylammoniumethersulfate, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.
Vorteilhaft zu verwendende amphotere Tenside sind
   1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
   2. N-Alkylaminosäuren, beispielsweise Aminopropylalkylglutamid, Alkylaminopropionsäure, Natriumalkylimidodipropionat und Lauroamphocarboxyglycinat.
Vorteilhaft zu verwendende nicht-ionische Tenside sind
   1. Alkohole,
   2. Alkanolamide, wie Cocamide MEA/DEA/MIPA,
   3. Aminoxide, wie Cocoamidopropylaminoxid,
   4. Ester, die durch Veresterung von Carbonsäuren mit Ethylenoxid, Glycerin, Sorbitan oder anderen Alkoholen entstehen,
   5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
   6. Sucroseester, -Ether
   7 Polyglycerinester, Diglycerinester, Monoglycerinester
   8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Kosmetische Zubereitungen im Sinne der vorliegenden Erfindung können auch als Gele vorliegen, die neben einem wirksamen Gehalt an der erfindungsgemäßen Wirkstoffkombination und dafür üblicherweise verwendeten Lösungsmitteln, bevorzugt Wasser, noch organische Verdickungsmittel, z. B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z. B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

Es wurden die folgenden Zusammensetzungen mit Phenoxyethanol hergestellt, die wahlweise auch mit Phenoxypropanol oder einem Gemisch von Phenoxyethanol und Phenoxypropanol hergestellt werden können:

| | |
|---|---|
| Undecylenamidopropyltrimethylammonium-Methosulfat | 1,0% |
| Phenoxyethanol | 2,0% |
| Pflegestoffe | 1,0% |
| Sodium Citrat | 0,45% |
| Wasser, ad | 100% |

| | |
|---|---|
| Undecylenamidopropyltrimethylammonium-Methosulfat | 5,0% |
| Phenoxyethanol | 2,0% |
| Alkylpolysaccharid | 3,0% |
| Rückfetter | 1,0% |
| Pflegestoffe | 2,0% |
| Sodium citrat | 0,3% |
| Wasser, ad | 100% |

| | |
|---|---|
| Undecylenamidopropyltrimethylammonium-Methosulfat | 4,0% |
| Phenoxyethanol | 2,0% |
| Alkylpolysaccharid | 1,0% |
| Cocosamidopropylbetain | 1,0% |
| Pflegestoffe | 4,0% |
| PEG-150 Distearate | 5,0% |
| Sodium citrat | 0,5% |
| Parfum | 0,08% |
| Wasser, ad | 100% |

## Patentansprüche

1. Wirkstoffkombinationen aus
a) Undecylenamidopropyltrimethylammonium-Methosulfat und
b) mindestens einem Arylalkylalkohol.

2. Kosmetische oder dermatologische Zubereitung, **dadurch gekennzeichnet, dass** sie 0,2 bis 15 Gew.-% einer Wirkstoffkombination aus Undecylenamidopropyltrimethylammonium-Methosulfat und mindestens einem Arylalkylalkohol enthält.

3. Zubereitung nach Anspruch 2, **dadurch gekennzeichnet, dass** sie als wässrige Formulierung oder als feste oder flüssige Waschlotion oder als Seife vorliegt.

4. Zubereitung nach einem der Ansprüche 2 oder 3, **dadurch gekennzeichnet, dass** sie 0,1 bis 10,0 Gew.-% Undecylenamidopropyltrimethylammonium-Methosulfat enthält.

5. Zubereitung nach einem der Ansprüche 2 bis 4, **dadurch gekennzeichnet, dass** sie 0,1 bis 5,0 Gew.-% eines oder mehrerer Arylalkylalkohole enthält.

6. Zubereitung nach einem der Ansprüche 2 bis 5, **dadurch gekennzeichnet, dass** zusätzlich Tenside und/oder Hilfs-, Zusatz- und/oder weitere Wirkstoffe enthalten sind.

7. Verwendung von Zubereitungen nach einem der Ansprüche 2 bis 6 zur antiseptischen Ganzkörperwaschung, zur antiseptischen Haarwäsche, zur antiseptischen Hand- oder Fußwäsche oder zur antiseptischen Intimwäsche.

8. Verwendung von Zubereitungen nach einem der Ansprüche 2 bis 6 zur Desinfektion von Haut, Händen und/oder Schleimhäuten verwendet werden.

## Claims

1. Active substance combinations of
a) undecylenamidopropyltrimethylammonium methosulphate and
b) at least one arylalkyl alcohol.

2. Cosmetic or dermatological preparation, **characterized in that** it comprises 0.2 to 15% by weight of an active substance combination of undecylenamidopropyltrimethylammonium methosulphate and at least one arylalkyl alcohol.

3. Preparation according to Claim 2, **characterized in that** it is in the form of an aqueous formulation or in the form of a solid or liquid washing lotion or in the form of a soap.

4. Preparation according to one of Claims 2 or 3, **characterized in that** it comprises 0.1 to 10.0% by weight of undecylenamidopropyltrimethylammonium methosulphate.

5. Preparation according to one of Claims 2 to 4, **characterized in that** it comprises 0.1 to 5.0% by weight of one or more arylalkyl alcohols.

6. Preparation according to one of Claims 2 to 5, **characterized in that** surfactants and/or auxiliaries, additives and/or further active substances are additionally present.

7. Use of preparations according to one of Claims 2 to 6 for antiseptic whole-body washing, for antiseptic hair washing, for antiseptic hand or foot washing or for antiseptic intimate washing.

8. Use of preparations according to one of Claims 2 to 6 for disinfecting skin, hands and/or mucosa are used.

## Revendications

1. Combinaison de substances actives constituées de:
a) méthosulfate d'undécylénamidopropyltriméthylammonium et
b) au moins un alcool arylalkylique.

2. Préparation cosmétique ou dermatologique, **caractérisée en ce qu'**elle contient de 0,2 à 15% en poids d'une combinaison de substances actives constituée de méthosulfate d'undécylénamidopropyltriméthylammonium et d'au moins un alcool arylalkylique.

3. Préparation selon la revendication 2, **caractérisée en ce qu'**elle se présente sous la forme d'une formulation aqueuse, comme lotion de lavage solide ou liquide ou comme savon.

4. Préparation selon l'une quelconque des revendications 2 ou 3, **caractérisée en ce qu'**elle contient de 0,1 à 10,0% en poids de méthosulfate d'undécylénamidopropyltriméthylammonium.

5. Préparation selon l'une quelconque des revendications 2 à 4, **caractérisée en ce qu'**elle contient de 0,1 à 5,0% en poids d'un ou plusieurs alcools arylalkyliques.

6. Préparation selon l'une quelconque des revendications 2 à 5, **caractérisée en ce qu'**elle contient en outre des agents tensioactifs et/ou des substances auxiliaires, des additifs et/ou d'autres substances.

7. Utilisation des préparations selon l'une quelconque des revendications 2 à 6 pour le lavage antiseptique de l'ensemble du corps, pour le lavage antiseptique des cheveux, pour le lavage antiseptique des mains ou des pieds ou pour le lavage antiseptique des parties intimes.

8. Utilisation des préparations selon l'une quelconque des revendications 2 à 6 pour la désinfection de la peau, des mains et/ou des muqueuses.
